# EUROPEAN PATENT SPECIFICATION

(11) **EP 1 658 384 B1**
(45) Date of publication and mention of the grant of the patent: **06.10.2010**
(21) Application number: 04786212.3
(22) Date of filing: 17.08.2004
(51) Int. Cl.: C12Q 1/68

(54) **METHOD FOR EARLY DETECTION AND MONITORING OF DISEASES BY ANALYSIS OF CELL-SURFACE-BOUND NUCLEIC ACIDS**
METHODE ZUR DETEKTION UND BEOBACHTUNG VON KRANKHEITEN DURCH ANALYSE VON AN DEN ZELLEN GEBUNDENEN NUKLEINSÄUREN
PROCEDE DE DETECTION PRECOCE ET DE SURVEILLANCE DE MALADIES PAR L'ANALYSE D'ACIDES NUCLEIQUES LIES A LA SURFACE DES CELLULES

(30) Priority: 18.08.2003 RU 2003125486
(43) Date of publication of application: 24.05.2006
(73) Proprietor: Universitätsklinikum Schleswig-Holstein, 23538 Lübeck (DE)
(72) Inventor: SCZAKIEL, Georg, 23627 Gross-Grönau (DE); VLASSOV, Valentin, Novosibirsk, 630090 (RU); LAKTIONOV, Pavel, Novosibirsk, 630060 (RU); RYKOVA, Elena, Novosibirsk, 630060 (RU); TAMKOVIC, Svetlana, Novosibirsk, 630117 (RU); SKVORTSOVA, Tat'yana, Novosibirsk, 630090 (RU)
(74) Representative: Biehl, Christian
(86) International application number: PCT/EP2004/009218
(87) International publication number: WO 2005/017197

(56) References cited:
- DE-A1- 3 721 400
- US-A1- 2003 143 600
- VASYUKHIN V ET AL: "K-RAS POINT MUTATIONS IN THE BLOOD PLASMA DNA OF PATIENTS WITH COLORECTAL TUMORS" CHALLENGES OF MODERN MEDICINE, ROME, IT, 1994, pages 141-150, XP001064078 ISSN: 1124-1357
- LEFORI L ET AL: "POINT MUTATIONS OF THE K-RAS GENE PRESENT IN THE DNA OF COLORECTAL TUMORS ARE FOUND IN THE BLOOD PLASMA DNA OF THE PATIENTS" PROCEEDINGS OF THE ANNUAL MEETING OF THE AMERICAN ASSOCIATION FOR CANCER RESEARCH, NEW YORK, NY, US, vol. 36, 1995, page A3319, XP001064064 ISSN: 0197-016X

## Description

The invention belongs to the field of diagnostic medicine and therapy monitoring. It is based on the development of non-invasive methods for early detection of human diseases including pre-cancerous states, early stages of cancer development, pathologies of pregnancy, monitoring of efficacy of anticancer therapy, etc.

It is an object of the invention to provide a method of early detection and monitoring of diseases. It is also an object of the invention to provide a method for the purpose of early detection and monitoring of diseases that is non-invasive. It is another object of the invention to provide a method that allows for the early detection of cancer of different genesis. It is furthermore an object of the invention to provide a method for the early detection in the pathology of pregnancy and it is an object of the invention to provide a method for the monitoring of the efficacy of anticancer therapy.

The method according to the invention is based on the investigation of cell-surface-bound extra-cellular nucleic acids from human blood. Blood samples are divided into plasma and cellular fractions. The cellular fraction is further subdivided into leucocytes and erythrocytes. Cell-surface-bound extra-cellular nucleic acids are eluted from cell surface with PBS-EDTA or by treatment of cells with trypsin solution. Eluted nucleic acids are isolated with and analysed for the presence of at least two specific markers (nucleotide sequences) associated with the disease or parameter of interest by using analytical methods such as PCR, multiplex PCR, hybridisation assay or other methods of investigation of specific sequences of nucleic acids.

The method enables one to increase the reliability and sensitivity of early detection of diseases or therapeutic schemes. This strategy shows improved sensitivity of the detection of specific DNA and RNA sequences in the fraction of nucleic acids associated with cell surface of blood cells when compared with nucleic acids isolated from the plasma fraction. This is especially important for the reliable detection of early stages of pathological processes at which the most part of nucleic acids circulating in the blood are associated with cell surface of blood cells. It is important to note that this methodology is non-invasive and, thus, the potential risk by the diagnosis itself is substantially minimized when compared to invasive methods.

The method according to the invention allows the isolation of nucleic acids, obtained from the cell-surface of blood-circulating cells, as diagnostic markers:
1. A patient's blood sample is being separated into plasma and cellular fraction.
2. The cellular fraction is further divided into erythrocytes and leukocytes.
3. Nucleic acids associated with the cell surface of leukocytes are eluted by treatment with PBS-EDTA.
4. The eluted nucleic acids are isolated by methods known in the state of the art (e.g. with a kit from Qiagen or any other suitable laboratory protocol).
5. The composition of these nucleic acid preparations and the absolute and relative amounts can be analysed with any suitable method known in the state of the art, e.g. PCR, Multiplex-PCR, hybridisation and sequencing methods.

In more detail the method of early diagnosis of diseases induced by abnormal functioning of cellular genomes comprises sampling of blood, dividing the blood into plasma and cellular fractions, isolating extracellular nucleic acids (exNA), revealing specific sequences of nucleic acids by means of polymerase chain reaction with subsequent analysis of the presence or absence of specific sequences in total PCR products, which differ from existing methods by the fact that cell-surface-bound extracellular nucleic acids are used as a source of extracellular nucleic acids instead of exNA isolated from plasma fraction, whereby the cellular fraction is divided into leucocytes and erythrocytes, cell-surface-bound extracellular nucleic acids are subsequently eluted from cell surface, exNA are isolated from eluates and these exNA are used for analysis of at least two specific sequences of exNA distinctive for the disease.

In a preferred embodiment of the invention the cell-surface-bound nucleic acids are eluted by treating the cells with 10 volumes of PBS with 5 mM EDTA at 4 °C with subsequent pelleting of the cells by centrifugation and collection of the supernatant, followed by the elution with 0,25 % trypsin solution, subsequent inactivation of the enzyme with trypsin inhibitor, centrifugation and collection of the supernatant.

### Description of the tables

The tables enclosed in the description summarize the results of an analysis using the method according to the invention and underline the impact of such method on the clinical diagnosis of diseases.
Table 1 shows the correlation between the symptom lung cancer and increased amounts of extra-cellular and cell-surface-bound nucleic acids (from leukocytes and from erythrocytes). Both groups were sampled from a lung cancer risk group and healthy donors. The number in percentages show to which extent the samples were marked positive for the gene-markers "APC" and "RASSF1A". The cell-surface-bound nucleic acids are divided into subcellular fractions for erythrocytes and leukocytes and further distinguished by their method of elution (PBS-EDTA or trypsin treatment).
Table 2 shows that the nucleotide sequences "c-myc" and "c-erbB2" are detectable in preparations of cell-surface-bound nucleic acids of leukocytes and erythrocytes in 9 % of patients with breast cancer.
Table 3 shows that the nucleotide sequences CK19" and "CEA are detectable in preparations of cell-surface-bound nucleic acids of leukocytes and erythrocytes as well as in cell-free plasma-DNA in a colon cancer risk group.
Table 4 shows an increase of DNA-concentration in plasma and of cell-surface-bound DNA of leukocytes and erythrocytes in samples of pregnant women with differing degree of pre-eclampsia.

**Table 1. Frequency of APC and RASSF1A genes promoter methylation in patients of lung cancer risky group and in healthy donors.**

| Analysed groups of patients | Gene | Year | exDNA in blood plasma | Cell-surface-bound exDNA | | | |
|---|---|---|---|---|---|---|---|
| | | | | Eluted from erythrocytes | | Eluted from leucocytes | |
| | | | | PBS/EDTA | Trypsin | PBS/EDTA | Trypsin |
| Lung cancer risk group, N=24 | APC | 1999 | 12,51% | 41,7% | 37,53% | 20,85% | 16,68% |
| | | 2000 | 16,68% | 37,53% | 21,19% | 12,51% | 0 |
| | RASSF1A | 1999 | 16,68% | 50,04% | 41,7% | 25,02% | 16,68% |
| | | 2000 | 20,85% | 41,7% | 21,19% | 20,85% | 8,34% |
| | B 1999-2003 - lung sarcoma was found in 62,5% patients from risk group, furthermore all patients with high level of promoter methylation were in that group | | | | | | |
| Healthy donors, N=21 | APC | 1999 | 0 | 4,17% | 0 | 4,17% | 0 |
| | | 2000 | 4,17% | 0 | 0 | 0 | 0 |
| | RASSF1A | 1999 | 0 | 0 | 0 | 0 | 0 |
| | | 2000 | 0 | 0 | 0 | 0 | 0 |

**Table 2. Frequency of c-myc and c-erbB2 expression in patients with breast cancer (N= 32).**

| Gene | Plasma DNA | Cell surface bound DNA | |
|---|---|---|---|
| | | Erythrocytes | Leucocytes |
| c-myc | 0 | 9% | 9% |
| c-erbB2 | 0 | 9% | 9% |

**Table 3. Frequency of CK19 and CEA genes expression in patients of colon cancer risky group and in healthy donors.**

| Analysed patients | Gene | Plasma DNA | Cell-surface-bound DNA | |
|---|---|---|---|---|
| | | | Erythrocyte | Leucocytes |
| Risks group, N=38 | CK19 | 26,31% | 60,53 % | 52,63% |
| | CEA | 23,68% | 57,89% | 47,37% |
| Control group, N=20 | CK19 | 15% | 20% | 15% |
| | CEA | 20% | 15% | 15% |

**Table 4. Quantitative analysis of fetal and total extra-cellular DNA levels in normal pregnancies and pregnancies with pre-eclampsia.**

| Checkup groups | Pregnancies with male fetus | Source of DNA | Levels of plasma DNA (genome equivalent per ml of maternal blood) | Levels of cell surface bounded DNA (genome equivalent per ml maternal blood) | |
|---|---|---|---|---|---|
| | | | | Erythrocyte | Leucocytes |
| Control (N=48) | 45 | fetal | 308,5* | 462 | 349 |
| | | | (0-1537) | (0-2396) | (0-1876) |
| | | total** | 22342,8 | 30352,5 | 24676 |
| | | | (400-226453) | (572-34269,7) | (409-27887,9) |
| Pregnancies with pre-eclampsia. (N=24) | 20 | fetal | 805,3 | 1046,5 | 887,76 |
| | | | (0-3864,8) | (0-5468,8) | (0-4567,9) |
| | | total | 63113,3 | 97771,5 | 80976,78 |
| | | | (1224-455545,8) | (1843,4-694989) | (1456-60435) |
| Pregnancies with severe pre-eclampsia. (N=8) | 8 | fetal | 3028,65 | 4481,44 | 3564 |
| | | | (347-6995,4) | (453-9648,96) | (407-7869,7) |
| | | total | 217786 | 328534,5 | 287654,8 |
| | | | (1324-744545) | (1934,3-1100592) | (1675-978657,7) |

| | | | | | |
|---|---|---|---|---|---|
| * average value ** both maternal and fetal extracellular DNA | | | | | |

## Claims

1. Method of early diagnosis of diseases induced by abnormal functioning of cellular genomes, comprising sampling of blood, dividing the blood into plasma and cellular fractions, isolating extracellular nucleic acids (exNA), revealing specific sequences of nucleic acids by means of polymerase chain reaction with subsequent analysis of the presence or absence of specific sequences in total PCR products, which differ from existing methods by the fact that cell-surface-bound extracellular nucleic acids are used as a source of extracellular nucleic acids instead of exNA isolated from plasma fraction, whereby the cellular fraction is divided into leucocytes and erythrocytes, cell-surface-bound extracellular nucleic acids are subsequently eluted from cell surface, exNA are isolated from eluates and these exNA are used for analysis of at least two specific sequences of exNA distinctive for the disease.

2. Method according to claim 1, **characterized by** a two-stage elution of exNA from the surface of leucocytes comprising the steps of eluting exNA by treatment of cells with 10 volumes of PBS supplied with 5mM EDTA at 4 °C, with subsequent pelleting of cells by centrifugation and collection of supernatant followed by elution of exNA with 0,25 % trypsin solution, with subsequent inactivation of the enzyme with trypsin inhibitor, centrifugation and collection of the supernatant.

3. Method according to claim 1, **characterized in** isolating the exNA by increased glass-milk protocol.

4. The use of the method according to claim 1 for early detection of cancer and / or pathologies of pregnancy.

## Patentansprüche

1. Verfahren zur Frühdiagnose von durch abnormes Funktionieren zellulären Genoms hervorgerufenen Krankheiten, aufweisend: Entnehmen einer Blutprobe, Aufteilen des Bluts in Plasma und Zellfraktionen, Isolieren von extrazellulären Nukleinsäuren (exNA), Aufzeigen spezifischer Nukleinsäuresequenzen mittels Polymerasekettenreaktion mit anschließender Analyse des Vorhandenseins oder Fehlens spezifischer Sequenzen im Gesamt-PCR-Produkt, die sich von bestehenden Verfahren **dadurch** unterschiedet, dass zelloberflächengebundene extrazelluläre Nukleinsäure als Quelle der extrazellulären Nukleinsäuren verwendet werden anstelle von aus der Plasmafraktion isolierten exNA, wobei die Zellfraktion in Leukozyten und Erythrozyten unterteilt ist, die zelloberflächengebundenen extrazellulären Nukleinsäuren anschließend von der Zelloberfläche eluiert werden, exNA aus den Eluaten isoliert und diese exNA zur Analyse von wenigstens zwei spezifischen Sequenzen von exNA, die für die Krankheit charakteristisch sind, verwendet werden.

2. Verfahren nach Anspruch 1, **gekennzeichnet durch** eine zweistufige Eluierung von exNA von der Oberfläche der Leukozyten, mit den Schritten: Eluieren von exNA durch Behandlung von Zellen mit 10 Volumenteilen PBS versetzt mit 5 mM EDTA bei 4 °C, mit darauffolgendem Pelletieren der Zellen **durch** Zentrifugation und Sammeln des Überstands, gefolgt von Eluierung der exNA mit 0,25 % Trypsinlösung, mit anschließender Inaktivierung der Enzyme mit Trypsininhibitor, Zentrifugation und Sammeln des Überstands.

3. Verfahren nach Anspruch 1, **gekennzeichnet durch** Isolieren der exNA **durch** das erweiterte "Glass-Milk"-Protokoll.

4. Verwendung des Verfahrens nach Anspruch 1 zur Früherkennung von Krebs und/oder Schwangerschaftspathologien.

## Revendications

1. Méthode de diagnostic précoce des maladies provoquées par le fonctionnement anormal du génome cellulaire comprenant le prélèvement de sang, la division du sang en plasma et en fractions cellulaires, l'isolement des acides nucléiques extracellulaires, la découverte de séquences spécifiques d'acides nucléiques au moyen d'une réaction en chaîne par polymérase avec une analyse ultérieure de la présence ou de l'absence de séquences spécifiques dans les produits de PCR totaux qui diffère des méthodes existantes dans la mesure où les acides nucléiques extracellulaires liés à la surface des cellules sont utilisés comme source d'acides nucléiques extracellulaires et non comme acides nucléiques extracellulaires isolés de la fraction plasmatique, où la fraction cellulaire est divisée en leucocytes et en érythrocytes, les acides nucléiques extracellulaires liés à la surface des cellules sont par la suite élués de la surface cellulaire, les acides nucléiques extracellulaires sont isolés des éluats et utilisés pour analyser au moins deux séquences spécifiques d'acides nucléiques extracellulaires caractéristiques de la maladie.

2. Méthode selon la revendication 1, **caractérisée par** une élution en deux étapes des acides nucléiques extracellulaires de la surface des leucocytes : élution des acides nucléiques extracellulaires par traitement des cellules avec 10 volumes de PBS mélangés à 5 mM d'EDTA à 4 °C, avec l'agglomération ultérieure des cellules par centrifugation et collecte du surnageant et élution des acides nucléiques extracellulaires au moyen d'une solution de trypsine à 0,25 %, avec l'inactivation ultérieure de l'enzyme avec l'inhibiteur de trypsine, la centrifugation et la collecte du surnageant.

3. Méthode selon la revendication 1, **caractérisée par** l'isolement des acides nucléiques extracellulaires grâce au protocole « glass-milk » (suspension de billes de silice) étendu.

4. L'utilisation de la méthode selon la revendication 1 pour une détection précoce du cancer et/ou des pathologies de la grossesse.
